# EUROPEAN PATENT SPECIFICATION

(11) **EP 2 810 645 B1**
(45) Date of publication and mention of the grant of the patent: **24.05.2017**
(21) Application number: 13170905.7
(22) Date of filing: 06.06.2013
(51) Int. Cl.: A61K 9/70, A61K 31/445

(54) **New Drug Delivery System**
Neues Arzneimittelabgabesystem
Nouveau système de distribution de médicaments

(43) Date of publication of application: 10.12.2014
(73) Proprietor: Sarac, A. Sezai, 34718 Istanbul (TR); Genctürk, Asli, Istanbul (TR); Duran Ozel, Arzu, Istanbul (TR)
(72) Inventor: Sarac, A. Sezai, 34718 Istanbul (TR); Genctürk, Asli, Istanbul (TR); Duran Ozel, Arzu, Istanbul (TR)
(74) Representative: Mutlu, Aydin

(56) References cited:
- WO-A2-2004/014304
- WO-A2-2006/106514
- WO-A2-2008/021113
- US-A1- 2010 297 238
- US-A1- 2011 229 551

## Description

### FIELD OF THE INVENTION

The present invention relates to drug delivery systems comprising nanofibers obtained from a polymer solution including pharmaceutical active ingredients and new fields of application thereof. The invention particularly relates to the use of donepezil comprising nanofibers for transdermally treating Alzheimer's disease.

### BACKGROUND OF THE INVENTION

Various drugs and drug combinations have been suggested for the treatment of Alzheimer type dementia. A great quantity of these drugs is those classified as acetylcholinesterase inhibitor. Having the following formula approved by FDA in 1996, Donepezil is one of the suggested and commonly used AchE inhibitors in various antidementia drug formulations.

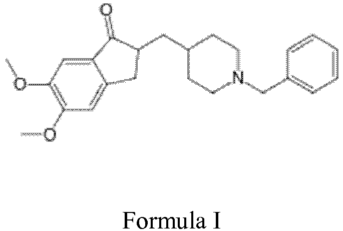

Donepezil is marketed in hydrochloride salt form and in tablet form for oral use with the trade name Aricept®. Donepezil formulations in prior art are mostly presented in oral dosage forms (tablet, capsule, granule etc.) as explained, for instance, in document numbered EP 1 878 444 A1. Nevertheless, many side effects have been reported associated with the oral use of donepezil. For instance, it has been known that side effects such as nausea, diarrhea, insomnia, vomiting, muscle spasms and many others may appear in the patients taking the first dose. The most common side effect of donepezil apart from these are gastrointestinal ailments, particularly high gastric acid secretion caused by cholinergic activity developed through gastrointestinal tract.

One of the solutions to alleviate these and similar side effects aims at decreasing the frequency of drug uptake by providing donepezil oral formulations in delayed release forms, thereby increasing patient compliance. While alleviating the side effects, these kinds of dosage regimen do not sufficiently eliminate them in entirety.

As a solution to the technical problems presented above, alternative dosage forms of donepezil such as ointment, injection, inhalant, gel and patch have been suggested. For instance, USpatent application no. 2010/0080842 A1 explains an adhesive composition applied on skin to realise transdermal donepezil release. However, the fact that topical applications are incapable of providing necessary drug dosage in desired periods of time and that it is hard to control the release amount stand out as crucial problems.

Due to the reasons presented, the main objective of the present invention is to provide a new transdermal form of donepezil intended for solution of the technical problems above. In the preparation of the transdermal pharmaceutical compositions of the invention, a mixture of polymer/ active ingredient in nanofiber form is foreseen; whereas the said nanofibers are preferably obtained with electrospinning method.

The technique involving use of a polymer and drug pair may include processes, such as complexing, conjugation and micelle formation. In order to obtain the polymer in nano structure, mold copying, colloidal lithography, interfacial polymerization, nanoprecipitation, multiple solvent emulsion evaporation, nano-identification, electrospray and electrospinning methods may be used.

Within the scope of the invention, electrospinning method is specifically preferred for obtaining nanofibers. This method is advantageous in obtaining broadened surface area and increased porousness transdermal dosage forms, whereas the method is also advantageous in terms of providing active ingredient/polymer mixture in optimum homogeneity and obtaining proper sized nanofibers with controllable sizes.

### BRIEF DESCRIPTION OF THE FIGURES

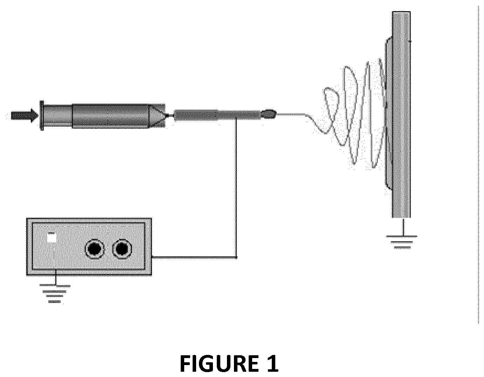

Figure 1 is a representative drawing showing the device used for the electrospinning method.
Figure 2 shows FTIR-ATR spectrum of polyurethane fiber containing Polyurethane and Donepezil.
Figure 3 is a graphic showing time-dependent release of donepezil from polyurethane fiber.

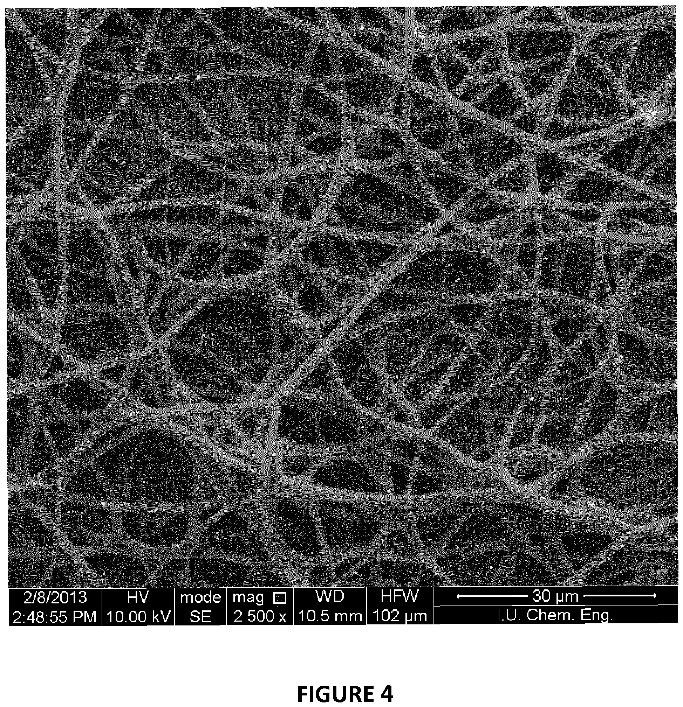

Figure 4 is a scanning electron microscope image of polyurethane fiber.

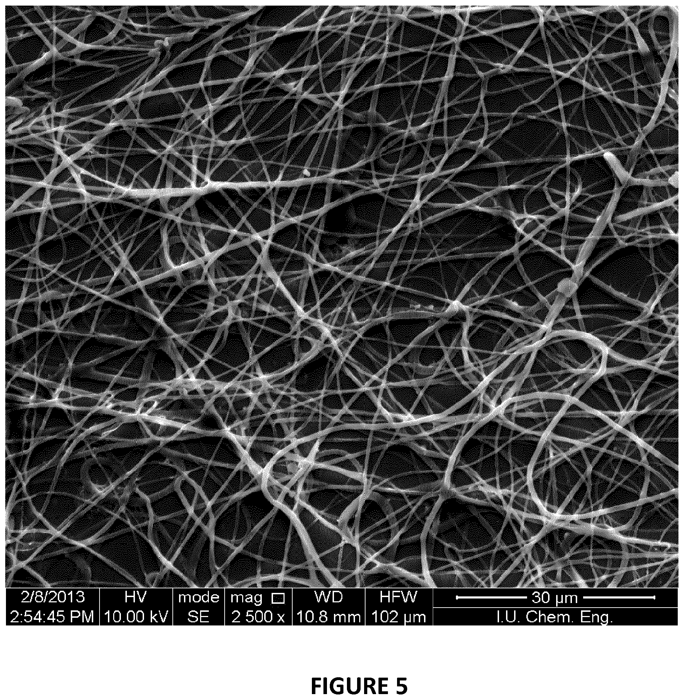

Figure 5 is a scanning electron microscope image of polyurethane fiber containing donepezil.

### DETAILED DESCRIPTION OF THE INVENTION

The present invention relates to the dosage forms of polymeric nanofibers comprising donepezil suitable for transdermal therapeutic applications and a process for preparingthe said nano fibers.

The system of the invention comprising therapeutic active ingredient may be obtained with the method of spin coating, electrospinning, electrospray or a method selected from the group containing them. The inventors of the invention have found out that the most advantageous way of manufacturing nanofibers offering high surface area in terms of transdermal application has been achieved through the electrospinning method.

Electrospinning method enables the formation of nanosized fiber from polymer with the aid of electric field forces. The main components of a basic electro production apparatus are;
1. High voltage power source,
2. Feeding unit (syringe, metal needle etc.)
3. Collector.

In this technique, polymer is dissolved in a proper solvent or melted with heat-treatment, put into a syringe or a glass pipette with a little hole on the tip. The pump, arranged at the back of the tube, creates a constant pressure that will push the polymer solution or fiber spinning solution through the pipette towards the tip. Afterwards, tension is applied between polymer solution and a collecting plate opposite the open end of the pipette. The applied electric may be potential alternative current or direct current or direct current based alternative voltage. Polymer drop hanging on the tip of the needle in feeding unit takes a round shape due to the forces applied by surface tension until a critical voltage value. At the moment that the applied potential difference reaches a threshold, electrostatic force eliminates the surface tension and polymer drop changes into a cone shape. This cone is named as Taylor Cone. Polymer solution without any surface tension flows through a specially designed and superfine jet nozzle towards the earthed target opposite the apparatus. The fibers piled up in earthened surface as fiber streak are continually pulled. By this electrospinning method, polymers turn into fibers by initially being charged with high voltage electric current and then flowing in the earthened plate through a nozzle (ABD patent No. 1.975.504, Seeram Ramakrishna, Kazutoshi Fujihara, Wee-Eong Teo, Teik-Cheng Lim & Zuwei Ma, "An Introduction To Electrospinning and Nanofibers")

The advantages of electrospinning method are that it is capable of manufacturing fibers/ particles in nanometer range, far smaller than ordinary fibers, that it has a high surface area/volume rate, that it has simple equipment requirement and that spin processing time is less than other spin processing time. Bulk properties of the substance affects bulk properties in nanometer scale and atomic properties become more effective. With the aid of electrospinning method, adjustable surface properties can appear.

According to an embodiment of the invention, nanofibers formed with donepezil and proper polymers for use as a transdermal drug system for treatment of Alzheimer type dementia are provided. Provision of donepezil-containing nanofibers in transdermal dosage forms for the treatment of Alzheimer's is not known in prior art.

According to another embodiment of the invention, in order to realize the transdermal use of the invention, specific transdermal systems suitable for the therapeutic use are presented.

According to another embodiment of the invention, processes related to the manufacture of the said therapeutic systems are provided.

The said transdermal therapeutic systems are preferably in patch or plaster form and are the pharmaceutical forms releasing drug at a predetermined constant speed and for a constant period peculiar to transdermal application. These systems may be in a patch or plaster form having 5-30 cm² suface area. Mainly 4 layers exist on approximately 1-2 mm transversal section of the plaster. (i) nonpermeable outer cover. (ii) Drug reservoir: contains drug in suspension, gel or silicon polymeric film. (iii) microporous permeable membrane controlling the rate: enables the release of the drug from the reservoir onto the skin with a constant speed and passive difusion. (iv) Adhesive layer: enables adhering to the skin and contains minor amount of drug. A thin plastic band may be placed under adhesive layer and it is disposed when the plaster is to be used.

Transdermal drug delivery system contains a number of physical care applications enabling the penetration to Stratum Corneum in order that many different active ingredients reach lower layers of the skin. Active ingredients show effectiveness and get results fastly without any side effects or damages to the skin.

Transdermal application particularly enables the release of drugs which may be eliminated by liver when taken orally. Transdermal drug delivery systems are advantageous compared to the other systems as it does not indicate the drawbacks and limitations of oral application, as it has higher treatment effectiveness, safety and patient compliance and is self-applicable by the patient. With transdermal use, better patient compliance and less side effects are acquired, thereby life quality of the patients are increased. It is known that it is hard to enable patient compliance in mental ailments such as Alzheimer's. Considering that the classic treatment processes require taking the necessary drug orally in particular hours of the day, it will be appreciated that it is necessitated to present transdermal systems providing long term release for Alzheimer's patients.

Polymeric biomaterials used in nanofibers of the invention are preferably biocompatible and/or biodegradable polymers and may be of natural and/or synthetic origin. Synthetic polymers may be selected from homopolymers, heteropolymers or copolymers and terpolymers. Nanofibers may be formed by use of one or a combination from the group of polyethylene (PE), polyurethane (PU), polycaprolactone, polycarbonate, polytetrafluoroethylene (PTFE), polyacetal (PA), polymethylmethacrylate (PMMA), polyethylene glycols, polyethylene terephthalate (PET), silicone rubber (SR), polysulfone (PS), polylactic acid (PLA), polyglycolic acid (PGA), poly(DTE carbonate), poly(propylene carbonate), polytetrafluoroethylene, polyacetal, polymethylmethacrylate, polyethylene terephthalate, polycaprolactone, polyvinylpyrrolidone, acrylic polymers and similar alpha hydroxy acid polyester, by the use of the one or a combination from the group beta-1,3-glucan, natural rubber, cellulose, Type-I collagen, Type-II collagen, alginate, gelatine, chitosan, fibrinogen, chitin, elastin, hydroxylapatite, dextran, laminin, hyaluronic acid, starch, silk and similar natural biopolymers and/or as a combination of natural/synthetic biopolymers. The polymer and polymer combinations used in the invention are not limited to those described hitherto.

Heteropolymer or copolymers of the invention are selected from alternative copolymer, periodic copolymer, statistical copolymer, block copolymer, terpolymer and graft copolymer. For instance; poly(lactic-co-glycolic acid), poly(ethylene glycol-co-lactide), poly(L-lactide-co-e-caprolactone), poly(ethylene-co-vinyl alcohol), styrenedivinylbenzene copolymer, poly(vinyl chlorite-co-vinyl acetate-co-vinyl alcohol), poly(ethylene-alt-maleic anhydride, polypropylene-graft-maleic anhydride), Polyethylene-graft-maleic anhydride, poly(maleic anhydride-alt-1-octadecene), poly(isobutylene-alt-maleic anhydride), polystyrene-block-poly(ethylene-ran-butylene)-block-polystyrene-graft-maleic anhydride. However, heteropolymer or copolymers that may be used in the invention are not limited thereto.

Acrylic polymers used in the invention are homopolymer, copolymer, terpolymer and acrylic acid-like polymers. Polyacrylates may be selected from the group containing acrylic acid, methacrylic acid, N-butyl acrylate, N-butyl methacrylate, hexyl acrylate, 2-ethylbutyl acrylate, isooctyl acrylate, 2-ethylhexyl acrylate, decyl acrylate, decyl methacrylate, dodecyl acrylate, dodecyl methacrylate, tridecyl acrylate, tridecyl methacrylate or mixtures thereof.

In this invention, solvent is used to dissolve the polymer and/or the drug. These are acetone, heptane, ethyl acetate, isopropanol, alcohol and/or derivatives thereof, ethylene glycol, hexane, toluene, xylene, dimethylformamide, chloroform, tetrahydrofurane, dichloromethane, acetic acid, hydrochloric acid, benzene and/or derivatives thereof, 1,4-dioxane, phosphate buffer salt solution, benzaldehyde, ethyl acetate, dietylether and/or derivatives thereof, toluene, n-hexane, acetic anhydride, acetonitrile, aniline, anisol, isobutyl methyl ketone, pyridine, dimethylsulphoxide, dimethylacetamide and water and/or mixtures thereof, however, they are not limited thereto.

In this invention, many additives may be added into polymer solution in order to decrease the surface tension or to develop characteristic properties of the solution or to obtain electrospun fiber with required properties. For example, Tritone, poloxamer, dodecylbenzene sulfonate, sodium dodecyl sulfate, sodium laurylsulfate may be added. Surfactants that may be used in the invention are not limited to the examples.

Cross linking agent that may be used in the invention is used to enhance mechanical property of the fiber. Materials such as ethylene glycol dimethacrylate, tetraethylene glycol dimethacrylate, divinylbenzene and divinyl acrylamide may be added as cross linking agents, however, cross linking agents used in the invention are not limited to the examples.

In this invention, type and concentration of the solvent may be selected depending on the chemical structure and molecular weight of the polymer. In the invention, polymer solution may contain approximately 1 to 40 wt%, and preferably 5 to 30 wt% of polymer solvent.

In this invention, polymer solution contains 5 to 40%, preferably 7 to 35%, and more preferably 10 to 15 % polymer by weight/volume.

In this invention, proper therapeutic active ingredient or therapeutic active ingredients are loaded into the polymer solution in liquid form. The concentration of the therapeutic active substance in polymer solution varies depending on the variety of the active ingredients used or the amount that shall take affect after being released from the system.

In a preferred embodiment of the invention, one or more drugs, having the antidementia and anticholinesterase activities selected from tacrine, rivastigmine, galanthamine, memantine and ginkgo biloba may be added as a supplement to donepezil in transdermal drug system. Drug combination is more preferably consisted of donepezil and memantine.

According to one preferred embodiment of the invention, the presented electrospun fiber composition may contain preferably 0.1 to 20 wt% of a therapeutically active ingredient or a mixture of pharmaceutical active ingredients.

In this invention, diameters of the fibers obtained with electrospinning method are between 1nm to 5 µm. Diameters of the fibers particularly preferred in the invention are between 1 nm and 2 µm, and more preferably between 1 nm and 1 µm.

According to one acpect of the invention a process, to produce the drug delivery system, comprises the following steps:
- preparation of a mixture comprising donepezil or pharmaceutically acceptable salt thereof and at least one polymer,
- obtaining nanofibers with diameters in the range of 1nm to 5 µm by applying a voltage to the said mixture with electrospinning method,
- applying the said nanofibers into a transdermal dosage form.

The electrospinning method in the invention is realized by applying a potential of 3 kV to 40 kV, preferably 5 kV to 35 kV more preferably 10 kV to 25 kV.

The distance between the syringe and collector in the applied electrospinning method is approximately 5 cm to 30 cm, preferably 5 cm to 25 cm, and more preferably 10 cm to 20 cm.

Polymer feeding rate of syringe changes depending on polymer and solvent properties in order that the fibers obtained from electrospinning process are smoother, uniform and bead-free. Polymer feeding rate of the syringe is at least 0.01 ml/min, preferably 0.1 ml/min, and more preferably 0.4 ml/min.

In this invention, electrospun fiber composition may be adjusted to release the therapeutic active ingredient time-dependently instantly or in a controlled manner. When fast release is desired, release within a few minutes intervals may be realized. With controlled release, delayed release for up to 24 hours can be enabled.

In this invention, electrospun fiber composition releases the therapeutically active ingredient slowly and time dependently. The release period of therapeutically active ingredient is 10 hours, preferably 20 hours, and more preferably 24 hours.

Excipients, polymers and solvents which will be used in the invention are explained below. However, these are not restrictive for this invention.

The pharmaceutical compounds for transdermal use in the present invention contain other additive or additives as well as pharmaceutically acceptable proper active ingredients. These kinds of compounds are the ones that may contain one or more excipients selected from the group comprising cross linking agents, plasticizers, anti-sticking agents, fillers, antioxidants, dispersants, binding agents, penetration-enhancing agents, surfactants, lubricants, glidants, diluents, protecting agents, viscosity-enhancing agents, antistatic agents, wetting agents, coloring agents, coating agents, solvents, softeners, emulgators, carriers, photo-protecting agents, thickening agents, gelling agents, microbial protecting agents, hardening agents, release control agents, opacifiers, emulsifiying agents, moisteners and stabilizers. However, these additives are not restictive for the present invention.

In this invention, the term "binding agent" refers to a substance or substance mixtures used to give volume to lower active dosage units and hold the ingredients together. As a binding agent; pregelatinized corn starch, pregelatinized starch, hydroxy propyl starch, gelatin, microcrystalline cellulose, cellulose, gums, polyvinylpyrrolidone, polymethacrylates, sodium carboxy methyl cellulose, starch, paraffins, stearic acid, methyl cellulose, ethyl cellulose, polyethylene glycol, magnesium aluminum silicate, carboxy-methylcellulose, hydroxy propylcellulose, hydroxy ethylcellulose, propylene glycol, polyoxyethylene-polypropylene copolymer, polyethylene ester, polyethylene sorbitan ester, polyethylene oxide, polysaccharides, poloxamers, alginic acids, collagen, albumin, crospovidone, povidone, copovidone, maltodextrin, hypromellose or mixtures thereof may be used.

In this invention, the term "dispersing agent" refers to substances enabling easy and fast dispersion of the dosage form in water. As a dispersing agent; agar agar, calcium carbonate, sodium carbonate, alginic acid, potato starch, corn starch, wheat starch, pregelatinized starch, starches such as sodium starch glycolate, microcrystalline cellulose, cross-linked poly vinyl pyrrolidone, sodium alginate, hydroxypropyl cellulose, cross-linked hydroxypropyl cellulose, croscarmellose sodium, clay, ion exchange resin, crospovidone, xylitol, D-sorbitol, D-mannitol, lactose, sucrose, urea, high molecular weight polymers, povidone, alginic acid, xanthan gum, colloidal silicon dioxide or mixtures thereof may be used.

In this invention, the term "surfactant" refers to a chemical compound affecting surface tension when dissolved in water or in aqueous solution. As a surfactant; polysorbates, sodiumlauryl sulfate, sodyumstearil fumarate, nonionic polyoxyethylene polyoxpropylene copolymer, hexadecyl trimethyl ammonium bromide, alkyl polyethylene oxide, poloxamers, octyl glucoside, sugar esters and glycerides of fatty acids, dodecyl betaine, dodecyl dimethylamine oxide, polyoxyl stearate, sodium stearate, polyethylene glycols, L-leucine, alkyl benzene sulfonate, fatty acids, quaternary ammonium compounds, sugar esters and glycerides of fatty acids or mixtures thereof may be used.

In this invention, the term "protective agent" refers to substances enabling the protection of water and water-soluble substances, and fat and fat-soluble substances against microorganisms. As a protective agent; 2-phenoxyethanol, sodium benzoate, benzoic acid, benzyl alcohol, ethylenediaminetetraacetic acid, sodium methyl parahydroxy benzoate, sodium propyl para hydroxy benzoate, sorbic acid, potassium sorbate, benzethonium chloride, chlorocresol, benzalkonium chloride, butyl paraben, methyl paraben, propylparaben, ethyl paraben, butyl hydroxy anisole (BHA), butyl hydroxy toluene (BHT), calcium acetate, citric acid, disodium edetate, glycerin, propyl gallate, sodium bisulfite, sodium citrate, sodium metabisulphite, boric acid, sorbic acid, sodium propionate, propylene glycol or mixtures thereof may be used.

In this invention, the term "viscosity-increasing agent" refers to an agent or agent mixtures enabling slow flow by thickening the liquid. As a viscosity enhancing agent; xanthan gum, guar gum, acacia, povidone, alginic acid, ethyl cellulose, gelatin, hydroxyethyl cellulose, hydroxypropyl cellulose, cetyl alcohol, polyvinyl pyrrolidone, hydroxy propyl methyl cellulose, polydextrose, carrageenan, methyl cellulose, sucrose, sorbitol, xylitol, hydroxypropyl methylcellulose, polyvinyl alcohol, ketearil alcohol, colloidal silicon dioxide or mixtures thereof may be used.

In this invention, as a solubility increasing agent; sodium caseinate, polysorbate, methacrylic acid copolymer or mixtures thereof may be used.

In this invention, the term "antistatic agent" refers to a substance or substance mixtures to decrease or eliminate the static electric in the content. As the said antistatic agent; long chain aliphatic amines and amides, quarterner amonium salts, phosphoric acid esters, polyethylene glycol esters, polyols, mono and diglyceride, fatty acid esters or mixtures thereof may be used.

In this invention, the term "wetting agent" refers to the substances used with the purpose of aiding easy dispersion in dispersion environment of hydrophobic drugs. As a wetting agent; sodium lauryl sulfate, docusate sodium, polysorbates, sorbitan monolaurate, octoxynol-9, nonoxynol-10, poloxamers, sodium carboxymethyl cellulose, bentonite, benzalkonium chloride, tetradecyltrimethyl ammonium bromide, cetylpyridinium chloride, glyceryl monostearate, macrogol cetostearyl ether, sorbitan tristearate, aluminium magnesium silicate or mixtures thereof may be used.

In this invention, the term "coloring agent" refers to the substances enabling a good looking, and optical differentiation between two formulations. Coloring agents, include but are not limited to, iron oxide pigments such as yellow iron oxide and red iron oxide, β-Carotene, red beet powder, chlorophyll, tartrazine, yellow orange, quinoline yellow, erythrosine, titanium dioxide pigments, caramel or mixtures thereof may be used.

As a solvent; purified water, alcohols such as ethyl alcohol, methyl alcohol, isopropyl alcohol, butyl alcohol, acetone, diacetone, polyols, polyethers, esters, alkyl ketones, methylene chloride, methyl acetate, ethyl acetate, isopropyl acetate, castor oil, ethylene glycol monoethyl ether, diethylene glycol monobutyl ether, diethylene glycol monoethyl ether, dimethyl sulfoxide, dimethyl formamide, tetrahydrofurane, acetonitrile or mixtures thereof may be used.

In this invention, the term "softener" refers to substances disabling the evaporation of the water by forming a thin film layer on skin. As a softening material; petrolatum, solid petrolatum, liquid paraffine, sorbitol, glycerine, hydrocarbons, lanoline, wax, fatty acids, cetyl alcohol, octyldodecanol, caprylic/ capric triglyceride, cetyl stearyl alcohol, cacao oil, diisopropyl adipate, glycerine, polyhydric alcohols and polyether derivatives, polyhydric alcohol esters, glyceryl monooleate, glyceryl stearate, linoleic acid, oleic acid, polypropylene glycol-15 stearyl ether (PPG-15 stearyl ether), polyethylene glycol, alcohol ethers with polyoxyethylene glycol oil, polyoxypropylene stearyl ether, propylene glycol stearate, stearic asit, stearyl alcohol, phospholipids, lecithin, sterols, cholesterol, cholesterol fatty acid esters and amides, urea, glyceryl monostearate, isopropyl myristate, isopropyl palmitate, cetostearyl alcohol, dimethicone, mineral oils, white solid paraffine, cetearyl alcohol or mixtures thereof may be used. Furthermore, herbal softeners such as castor-oil, coconut oil, olive oil or herbal wax may also be used.

In this invention, the term "emulgator" refers to the substances enabling homogen dispersion among two immiscible liquid phases. As an emulgator; polyethylene glycol stearate, polysorbate, polyglyceryl oleate, polyoxyethylene lauryl ether, ethoxylated lanoline, stearyl alcohol, cetostearyl alcohol, macrogol cetostearyl, glyceryl monostearate, cetyl alcohol, polyoxyethylene lauryl alcohol, polyoxyethylene sorbitan monostearate, polyoxyethylene stearate, sorbitan monostearate, propylene glycol stearate, aluminum starch octenylsuccinate, ammonium hydroxide, a white beewax, a synthetic beewax, carbomer, cetearyl alcohol, cyclo-methicone, diglyceride, dimethicone, disodium monooleamido sulfosuccinate, pentaerythritol, glyceryl, glyceryl monooleate, glyceryl stearate, lanolin, magnesium hydrogen stearate, mineral oil, monoglycerides, polyethylene glycol, polyethylene glycol distearate, polyethylene glycol monocetyl ether, polyethylene glycol monostearate, polyoxyethylene glycol, polyoxyl cetostearyl ether, polyoxyl stearate, simethicone, sorbitan monolaurate, sorbitan monooleate, sorbitan monopalmitate, sorbitan palmitate, stearic acid, triethanolamine or sodium lauryl sulfate or mixtures thereof may be used.

In the invention, as a "carrier"; propylene glycol, purified water, castor oil, diisopropyl adipate, ethoxylated alcohol, fatty alcohol citrate, glycerine, hexylene glycol, isopropyl alcohol, isopropyl myristate, isopropyl palmitate, mineral oil, phosphoric acid, polyethylene terephthalate glycol, polyethylene glycol, polyethylene glycol monostearate, polyoxyl cetostearyl ether, polyoxypropylene stearyl ether, polysorbate, octyldodecanol, propylene carbonate, saturated fatty acid triglyceride, benzoic acid, ethanol or mixtures thereof may be used.

In this invention, the term "penetration enhancer" refers to an absorption enhancer. As a penetration enhancer; fatty acids or derivatives thereof, fatty acid esters, fatty alcohols, glycols and glycol esters, 1,3-dioxolane and 1,3-dioxans, macrocyclic ketones comprising at least 12 carbons, oxazolidinone and oxazolidinone derivatives, alkyl-2-(N,N-disubstituted amine)alkanoate ester, (N,N- disubstituted amine alkanole alkanoate, oleic acid, oleyl alcohol, cyclopentadecanone, propylene glycol monolaurate, 2-n-nonyl-1,3-dioxolane, dodecyl 2-(N,N-dimethylamino) propionate or salts thereof, octyl dimethyl para aminobenzoate, octyl-para methoxy cinamate or mixtures thereof may be used.

In this invention, the term "antioxidant" refers to the substances preventing oxidations caused by free radicals and having the capacity of catching and stabilizing free radicals. As an antioxidant; tocopherol (Vitamin E), ascorbic acid (Vitamin C), vitamins such as Vitamin A, Vitamin K, caratenoids, carotenes (for example; α-carotene, β-carotene, lycopene, lutein, zeaxanthin), minerals (Se, Zn), butyl hydroxy anisol (BHA), butyl hydroxy toluene (BHT), ethylgallate, propylgallate, dodecylgallate, taurine, organosulphur compounds (allium, alkyl sulfate, indoles), low molecule weight antioxidants (GSH-Px, uric acid) or mixtures thereof may be used.

In this invention, as a photo-protecting agent; iron oxide derivatives, metal oxides, titanium oxides or mixtures thereof may be used.

In this invention, the term "thickening agent" refers to the substances used to reinforce the formulations against various pressures and forces. As thickening agent; cetyl alcohol, aluminium stearate, dimethicone, cetearyl alcohol, arabic gum, gummi tragacanth, alginate, carrageenan, xanthan gum, guar gum, cetostearyl alcohol, cetyl ester wax, dextrine, glyceryl monostearate, hydroxypropyl cellulose, kaoline, polyethylene white petrolatum, propylene glycol stearate, starch, wax, white wax, bentonite, beewax, white beewax, synthetic beewax, paraffine, white solid paraffine, white soft paraffine, solid petrolatum, pectin, carbomer, polyvinylpyrrolidone or mixtures thereof may be used.

In this invention, "as gelling agent"; carbopol, carbomer, hydroxy propyl methyl cellulose, methyl cellulose, sodium carboxy methyl cellulose, polyacrilate polymers or mixtures thereof may be used.

In the invention, as a release controlling agent; polyvinyl acetate phthalate, polyethylene glycol- polyvinyl alcohol copolymer, polyacrylic acid derivatives, polysaccharide derivatives, methacrylate polymer, polymethacrylate, ethyl methacrylate copolymer, methacrylate acid-methylmethacrylate copolymer, methacrylate acid-ethyl acrylate copolymer, polylactic acid, polylactic acid copolymer, polyvinyl pyrrolidine, polyvinyl alcohol, glyceride, polyethylene oxide, glycerile behenate, methacrylic acid copolymers, hydroxypropyl methylcellulose, hydroxypropyl cellulose, hydroxypropyl methylcellulose acetate, dioxy methyl cellulose succinate, carboxy methyl ethyl cellulose, sodium carboxy methyl cellulose, ethyl cellulose, methyl acrylate, ethylacrylate, methylacrylate, ethylmethacrylate, acrylic and methacrylic acid esters, sodium alginate, hypromellose, hypromellose phthalate, hypromellose acetate succinate, cellulose butyrate phthalate, cellulose hydrogen phthalate, cellulose propionate phthalate, cellulose acetate phthalate, cellulose acetate trimellitate, gelatine, shellac, xantan gum or mixtures thereof may be used.

In this invention, the term "plasticizer" refers to an agent increasing the flexibility of pharmaceutical compounds and decreasing fracturing risk. They are supposed to be compatible with polymer and not be volatile. As a plasticizer; polyethylene glycols (Macrogol), glycerine, propylene glycol, acetyl citrate, amyl oleate, myristyl acetate, butyl oleate, butyl stearate, triacetine, diethyl phthalate, acetylated monoglycerides or mixtures thereof may be used.

In this invention, the term "moistening agent" refers to the substances regulating and controlling the moisture amount between preparate and air. As a moistening agent; glycerine, sorbitol, propylene glycol, urea, substances with collodial structure, liquid paraffine, petrolatum, liquid petrolatum, cellulose and substances with cellulose structure, gums (tragacanth), some electrolytes (A1 salts, mercury salt, borax) or mixtures thereof may be used.

In this invention, the term "stabilizer" refers to the substances preventing crystalization or phase separation when added. As a stabilizer; benzoic acid, edetic acid, salicylic acid, sorbic acid, sodium dehydroacetate, tocopherol, butylated hydroxyanisole, butylated hydroxytoluene, propylgallate, castor oil, oleyl alcohol, poloxamer and poloxamines (polyoxyethylene and polyoxypropylene block copolymer), xanthan gum, ethoxylated esters of sorbitan fatty acids, polysorbates such as polysorbate 80 or Tween 80, ethoxylated mono- and diglycerides, ethoxylated lipids, ethoxylated oil alcohols and oil acids, diacetyl phosphate, phosphatidyl glicerol, saturated or unsaturated fatty acids, sodium cholate, sodium glicholate, sodium taurocholate, paraoxybenzoic acid, ethylene diamine tetraacetic acid (EDTA), diethylene triamine penta acetic acid or mixtures thereof may be used.

The invention mainly relates to the use and obtaining of a drug delivery system comprising fibers obtained with electrospinning method from a polymer solution containing suitable pharmaceutically acceptable active ingredients and /or pharmaceutically acceptable derivatives thereof and/or pharmaceutically acceptable and suitable excipients.

The composition of the invention may be applied on one or more places on the skin selected from the group of foot, ankle, leg, hand, palm, arm, armpit, back, breast, venter, hip, inguen, head, forehead and the like. The composition shall preferably be applied to an area providing maximum systemic absorption due to the rise of blood flow and temperature.

Surprisingly, as the dimension of the fibers formed with electospinning method decreases, surface area per volume increases, thereby mechanical performance of the fiber is increased and fiber forms that may be used in many important applications are created. Accordingly, diameters of the nanofibers in transdermal composition to realise the purpose of the invention are between 1nm and 5 µm in the invention. Diameters of the fibers in the invention are preferably between 1 nm and 2 µm, more preferably between 1nm and 1 µm.

The inventors have also found out that when the polymer used in nanofiber compound is polyurethane, the invention has further technical advantages. This specific polymer as specified in examples below has affected the mass transfer rate of the required drug dosage positively when particularly long-term release is aimed. The explanations related to them are present in the examples below.

The drug-containing polymer structure obtained in the current invention was characterized with FTIR-ATR Spectroscopy, Visible and UltraViolet Spectroscopy, Dynamic Mechanical Analysis, Atomic Force Microscobe, Scanning Electron Microscobe and Electrochemical Impedence Spectroscopy.

The examples below showing exemplary embodiments of the present invention should not be regarded as restrictive.

### EXAMPLES

### Example 1

### Preparation of Polymer Fibers Free of Drugs

- In order to prepare a solution containing at least one polymer inbetween a range of 1 to 40 wt.%, polymer or polymer combination in proper amount is dissolved in a proper solvent or solvent mixture in room temperature (approximately 25 °C),
- Homogenous polymer solution is obtained by stirring within approximately a 1-7 hour interval,
- The obtained polymer solution is filled into a syringe; polymer drop hanging on the tip of the needle takes a round shape due to the forces applied by surface tension until a critical voltage value. At the moment that the applied potential difference reaches a threshold, electrostatic force eliminates the surface tension forces and polymer drop changes into a cone shape. Polymer solution without any surface tension flows through a specially designed and superfine jet nozzle towards the earthed target opposite the apparatus.

### Example 2

### Preparation of Polymer Fibers Containing Drugs

- In order to prepare a solution containing at least one polymer between the range of 1 to 40 wt.%, polymer or polymer combination in proper amount is dissolved in a proper solvent or solvent mixture in room temperature (approximately 25 °C),
- Homogenous polymer solution is obtained by stirring within approximately 1-7 hour interval,
- The obtained polymer solution is added at least 0.1% active ingredient or active ingredients and stirred in approximately a 10-60 minutes interval.
- The solution containing polymer or polymer combination and drug or drug combinations is filled into a syringe, and fibers containing active ingredient with electrospinning method are obtained.

**Parameters Used in Electrospinning Method**

| **Used Components** | **Flow rate (ml/hr.)** | **Applied Voltage (kV)** | **Distance (cm)** |
|---|---|---|---|
| [Polymer or polymer combination] | ~ 0.2-2 | ~ 5-25 | ~ 5-25 |
| [Polymer or polymer combination +Active substance(s)] | ~ 0.2-2 | ~ 5-25 | ~ 5-25 |
| [Polymer or polymer combination +Active substance(s) + Inactive substance(s) ] | ~ 0.2-2 | ~ 5-25 | ~ 5-25 |

### Example 3

### Preparation of Polyurethane Nanofibers Containing Donepezil:

- Approximately 10% polyurethane wt./vol. is dissolved in dimethylformamide by stirring for 3 hours at room temperature (approximately 25 °C)
- The obtained polymer solution is filled into a syringe and fiber is obtained with electrospinning method,
- The obtained polyurethane solution is added 2% Donepezil and stirred for 30 minutes.
- Polymer solution containing polyurethane and donepezil is filled into syringe and nanofibers containing active substance are obtained with electrospinning method.

**Parameters Used in Electrospinning Method**

| **Used Components** | **Flow Rate(ml/hr.)** | **Applied Voltage (kV)** | **Distance (cm)** |
|---|---|---|---|
| Polyurethane | ~ 0.5-1 | ~ 10-15 | ~ 10-15 |
| [Polyurethane + Donepezil] | ~ 0.2-2 | ~ 5-25 | ~ 5-25 |

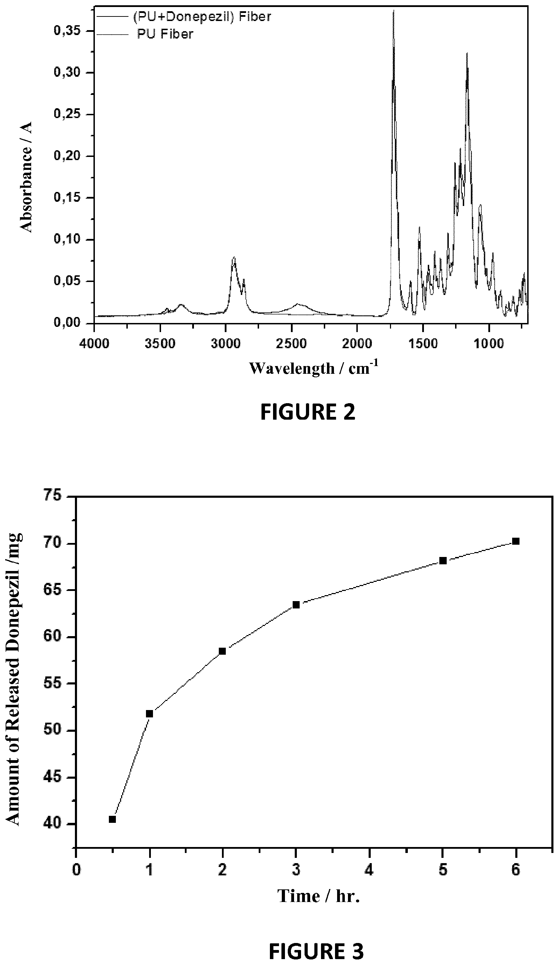

Dissolution rate of the obtained nanofibers is determined. In these studies, a system, which is stated in "dissolution test monograph for transdermal patch" (App. XII E) given by American pharmacopeia, was studied. In dissolution rate experiments, isotonic pH 6.5 phosphate buffer is used as a medium enabling donepezil hydrochloride infinite dilution (sink condition). The temperature is adjusted to 32 °C and rotational speed of the pallettes is adjusted to 50 cycles per minute. Stainless steel discs having 125 dm intervals are arranged on sections with 2,5 cm diameters taken from the formulations. This apparatus is arranged into a beaker of 500 ml. In predetermined time intervals (30^{th} minute, 1^{st}, 2^{nd} and 6^{th} hours), 2 ml samples are filtered from a membrane filter (0,45 dm). Donepezil hydrochloride amounts are calculated with ultra violet visible spectroscopy. Dissolution profiles of the formulations are created with active substance amounts. As shown in Figure 3, release rate of approximately 65 mg obtained within 4 hours from the beginning of the release has been maintained till 6^{th} hour. This test shows that the nanofibers presented within this invention can realize a consistent release as an alternative to oral dosage forms, therewithal may eliminate the disadvantages of conventional transdermal applications; and surprisingly, the stability of active ingredient is increased.

## Claims

1. A drug delivery system in a pharmaceutical transdermal patch or plaster form containing nanofibers having a diameter between 1 nm and 5 µm, comprising donepezil or a pharmaceutically acceptable salt thereof as active ingredient and a synthetic polymer of polyurethane for use in treatment of Alzheimer type dementia.

2. A drug delivery system for use according to Claim 1, wherein the said delivery system comprises an additional active ingredient selected from tacrine, rivastigmine, galanthamine, memantine, ginkgo biloba and pharmaceutically acceptable salts thereof.

3. A drug delivery system for use according to Claim 1, wherein the said nanofibers further comprise a solvent selected from acetone, heptane, ethyl acetate, isopropanol, alcohol and/or derivatives thereof, ethylene glycol, hexane, toluene, xylene, dimethylformamide, chloroform, tetrahydrofurane, dichloromethane, acetic acid, hydrochloric acid, benzene and/or derivatives thereof, 1,4-dioxane, phosphate buffer salt solution, benzaldehyde, ethyl acetate, dietylether and/or derivatives thereof, toluene, n-hexan, acetic anhydride, acetonitrile, aniline, anisol, isobutyl methyl ketone, pyridine, dimethylsulphoxide, dimethylacetamide and water and/or mixtures thereof.

4. A drug delivery system for use according to Claim 1, wherein the nanofiber composition comprises 0.1 to 20 % pharmaceutically active ingredient by weight.

5. A drug delivery system for use according to Claim 1, wherein the nanofiber composition comprises 1 to 40 % polymer by weight.

6. A drug delivery system for use according to the any of the preceding claims, wherein the said drug delivery system further comprises one or more excipients selected from the group comprising a binding agent, dispersant, filler, surfactant, lubricant, glidant, diluent, preservative, viscosity-increasing agent, solubility-increasing agent, antistatic agent, wetting agent, coloring agent, coating agent, solvent, softening agent, emulgator, carrier, antioxidant, photo-protecting agent, thickening agent, gelling agent, microbial protecting agent, hardening agent, release controlling agent, plasticizer, opacifier, moistening agent, stabilizer and cross linking agent.

7. A drug delivery system for use according to any of the preceding claims, wherein the nanofiber composition comprises a surface tension reducing agent.

8. A drug delivery system for use according to Claim 2, wherein said nanofibers comprise donepezil and memantine.

9. A drug delivery system for use according to Claim 1, wherein said nanofibers are electrospun fibers.

10. A drug delivery system for use according to Claim 1 and 9, wherein said nanofibers have diameters in the range of 1 nm to 2 µm.

11. A drug delivery system for use according to Claim 10, wherein said nanofibers have diameters in the range of 1 nm to 1 µm.

## Patentansprüche

1. Ein Arzneimittelabgabesystem in der Form eines transdermalen Patches oder Pflasters, enthaltend Nanofasern mit einem Durchmesser zwischen 1 nm und 5 µm, umfassend Donepezil oder ein pharmazeutisch verträgliches Salz davon als aktiven Inhaltsstoff und ein synthetisches Polyurethanpolymer zur Verwendung in der Behandlung von Alzheimer-typischer Demenz.

2. Ein Arzneimittelabgabesystem zur Verwendung nach Anspruch 1, wobei das Abgabesystem einen weiteren aktiven Inhaltsstoff, ausgewählt aus Tacrin, Rivastigmin, Galantamin, Memantin, Ginkgo und pharmazeutisch verträglichen Salzen davon, umfasst.

3. Ein Arzneimittelabgabesystem zur Verwendung nach Anspruch 1, wobei die Nanofasern weiter ein Solvens, ausgewählt aus Aceton, Heptan, Ethylacetat, Isopropanol, Alkohol und/oder Derivaten davon, Ethylenglykol, Hexan, Toluol, Xylol, Dimethylformamid, Chloroform, Tetrahydrofuran, Dichlormethan, Essigsäure, Chlorwasserstoffsäure, Benzol und/oder Derivate davon, 1,4-Dioxan, Phosphatgepufferte Salzlösung, Benzaldehyd, Diethylether und/oder Derivate davon, n-Hexan, Essigsäureanhydrid, Acetonitril, Anilin, Anisol, Methylisobutylketon, Pyridin, Dimethylsulphoxid, Dimethylacetamid und Wasser und/oder Mischungen davon, umfasst.

4. Ein Arzneimittelabgabesystem zur Verwendung nach Anspruch 1, wobei die Nanofaser-Komposition 0,1 bis 20 Gew.% pharmazeutisch aktiven Inhaltsstoff umfasst.

5. Ein Arzneimittelabgabesystem zur Verwendung nach Anspruch 1, wobei die Nanofaser-Komposition 1 bis 40 Gew.% Polymer umfasst.

6. Ein Arzneimittelabgabesystem zur Verwendung nach einem der vorhergehenden Ansprüche, wobei das Arzneimittelabgabesystem weiter einen oder mehrere Hilfsstoffe, ausgewählt aus der Gruppe umfassend ein Bindemittel, Dispergiermittel, Füllstoff, Tensid, Schmiermittel, Gleitmittel, Verdünnungsmittel, Konservierungsmittel, Viskositätserhöhungsmittel, Löslichkeitserhöhungsmittel, Antistatika, Benetzungsmittel, Färbemittel, Beschichtungsmittel, Lösemittel, Weichmacher, Emulgator, Trägersubstanz, Antioxidans, Lichtschutzmittel, Gelierungsmittel, mikrobielle Schutzmittel, Härtungsmittel, Trennmittel, Weichmacher, Opakmacher, Befeuchtungsmittel, Stabilisatoren und Vernetzungsmittel, umfasst.

7. Ein Arzneimittelabgabesystem zur Verwendung nach einem der vorhergehenden Ansprüche, wobei die Nanofaser-Komposition ein Mittel zur Verminderung der Oberflächenspannung umfasst.

8. Ein Arzneimittelabgabesystem zur Verwendung nach Anspruch 2, wobei die Nanofasern Donepezil und Memantin umfassen.

9. Ein Arzneimittelabgabesystem zur Verwendung nach Anspruch 1, wobei die Nanofasern elektrogesponnene Fasern sind.

10. Ein Arzneimittelabgabesystem zur Verwendung nach Anspruch 1 und 9, wobei die Nanofasern Durchmesser im Bereich von 1 nm bis 2 µm haben.

11. Ein Arzneimittelabgabesystem zur Verwendung nach Anspruch 10, wobei die Nanofasern Durchmesser im Bereich von 1 nm bis 1 µm haben.

## Revendications

1. Système d'administration de médicaments en forme de patch ou pansement transdermique pharmaceutique contenant des nanofibres présentant un diamètre compris entre 1 nm et 5 µm, comprenant du donepezil ou un sel pharmaceutiquement acceptable de celui-ci comme ingrédient actif et un polymère synthétique de polyuréthane pour l'utilisation dans le traitement d'une démence de type Alzheimer.

2. Système d'administration de médicaments pour l'utilisation selon la revendication 1, où ledit système d'administration comprend un ingrédient actif supplémentaire choisi parmi la tacrine, la rivastigmine, la galanthamine, la mémantine, le ginkgo biloba et leurs sels pharmaceutiquement acceptables.

3. Système d'administration de médicaments pour l'utilisation selon la revendication 1, où lesdites nanofibres comprennent en outre un solvant choisi parmi l'acétone, l'heptane, l'acétate d'éthyle, l'isopropanol, l'alcool et/ou ses dérivés, l'éthylène glycol, l'hexane, le toluène, le xylène, le diméthylformamide, le chloroforme, le tétrahydrofurane, le dichlorométhane, l'acide acétique, l'acide chlorhydrique, le benzène et/ou ses dérivés, le 1,4-dioxane, une solution tampon de sel de phosphate, le benzaldéhyde, l'acétate d'éthyle, le diéthyléther et/ou ses dérivés, le toluène, le n-hexane, l'anhydride acétique, l'acétonitrile, l'aniline, l'anisol, le méthylisobutylcétone, la pyridine, le diméthylsulfoxyde, le diméthylacétamide et l'eau et/ou des mélanges de ceux-ci.

4. Système d'administration de médicaments pour une l'utilisation selon la revendication 1, où la composition des nanofibres comprend 0,1 à 20% d'ingrédient pharmaceutiquement actif en poids.

5. Système d'administration de médicaments pour l'utilisation selon la revendication 1, où la composition des nanofibres comprend 1 à 40% de polymère en poids.

6. Système d'administration de médicaments pour l'utilisation selon l'une quelconque des revendications précédentes, où ledit système d'administration de médicaments comprend en outre un ou plusieurs excipients choisis dans le groupe comprenant un liant, un dispersant, une charge, un tensioactif, un lubrifiant, un agent glissant, un diluant, un conservateur, un agent augmentant la viscosité, un agent augmentant la solubilité, un agent antistatique, un agent mouillant, un agent colorant, un agent de revêtement, un solvant, un agent adoucissant, un émulgateur, un support, un antioxydant, un agent photo-protecteur, un agent épaississant, un agent gélifiant, un agent protecteur microbien, un agent durcissant, un agent de contrôle de libération, un plastifiant, un opacifiant, un agent mouillant, un stabilisant et un agent de réticulation.

7. Système d'administration de médicaments pour l'utilisation selon l'une quelconque des revendications précédentes, où la composition des nanofibres comprend un agent réducteur de tension de surface.

8. Système d'administration de médicaments pour l'utilisation selon la revendication 2, où lesdites nanofibres comprennent le donépézil et la mémantine.

9. Système d'administration de médicaments pour l'utilisation selon la revendication 1, où lesdites nanofibres sont des fibres électrofilées.

10. Système d'administration de médicaments pour l'utilisation selon la revendication 1 et 9, où lesdites nanofibres présentent des diamètres dans la plage de 1 nm à 2 µm.

11. Système d'administration de médicaments pour l'utilisation selon la revendication 10, où lesdites nanofibres présentent des diamètres dans la plage de 1 nm à 1 µm.
